(19) Europäisches Patentamt
European Patent Office
Office européen des brevets

(11) Veröffentlichungsnummer: **0 363 781 A1**

(12) **EUROPÄISCHE PATENTANMELDUNG**

(21) Anmeldenummer: **89118254.5**

(22) Anmeldetag: **02.10.89**

(51) Int. Cl.5: **C07C 303/06 , C07C 309/17**

(30) Priorität: **10.10.88 DE 3834393**

(43) Veröffentlichungstag der Anmeldung:
**18.04.90 Patentblatt 90/16**

(84) Benannte Vertragsstaaten:
**ES GR**

(71) Anmelder: **Henkel Kommanditgesellschaft auf Aktien**
**Postfach 1100 Henkelstrasse 67**
**D-4000 Düsseldorf 1(DE)**

(72) Erfinder: **Bernd Fabry, Dr.**
**Danziger Strasse 31**
**D-4052 Korschenbroich(DE)**

(54) **Verfahren zur Herstellung hellfarbiger alpha-Sulfofettsäurealkylestersalze.**

(57) Bei der Herstellung hellfarbiger alpha-Sulfofettsäurealkylestersalze durch Sulfonieren von Fettsäurealkylestern mit gasförmigem Schwefeltrioxid und nachfolgendes Aufarbeiten sulfoniert man die Fettsäurealkylester in Gegenwart von 0,25 bis 1,5 mol-% -mol-% - bezogen auf Fettsäurealkylester - mindestens eines Fettsäureamids der Formel I

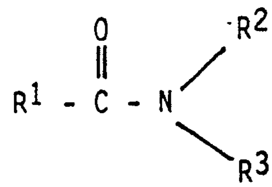

(I)

in der $R^1$ einen geradkettigen Alkylrest mit 5 bis 21 C-Atomen bedeutet, $R^2$ und $R^3$ abhängig voneinander ein Wasserstoffatom, einen geradkettigen oder verzweigten Alkylrest mit 1 bis 6 C-Atomen darstellen oder $R^2$ und $R^3$ zusammen eine Tetra- oder Pentamethylenkette eines heterocyclischen Ringsystems bilden, in dem gegebenenfalls eine Methylengruppe durch ein Sauerstoffatom, ein Schwefelatom oder eine NH-Gruppe ersetzt sein kann.

EP 0 363 781 A1

## Verfahren zur Herstellung hellfarbiger alpha-Sulfofettsäurealkylestersalze

Die Erfindung betrifft ein Verfahren zur Herstellung hellfarbiger alpha-Sulfofettsäurealkylestersalze durch Sulfonieren von Fettsäurealkylestern mit Schwefeltrioxid und nachfolgendes Aufarbeiten.

Die Sulfonierung von Fettsäurealkylestern zur Herstellung von alpha-Sulfofettsäurealkylestersalzen (nachfolgend als "Estersulfonate" bezeichnet) ist seit langem bekannt. Als technisches Ausgangsmaterial werden Fette und/oder Öle pflanzlichen oder tierischen Ursprungs verwendet, aus denen die Fettsäurealkylester entweder durch Fettspaltung und nachfolgende Veresterung mit niederen Alkanolen, insbesondere Methanol, oder durch Umesterung der natürlichen Triglyceride mit niederen Alkanolen erhalten werden. Die anfallenden Fettsäureestergemische enthalten - je nach Ursprung des natürlichen Rohstoffs - Ester von Fettsäuren eines vergleichsweise breiten Kettenlängenbereichs, der üblicherweise 6 bis 22 C-Atome umfassen kann. Derartige Fettsäureestergemische werden nach den bekannten Verfahren vorzugsweise mit gasförmigem Schwefeltrioxid sulfiert. Dies führt zu mehr oder weniger stark verfärbten, sauren Rohsulfonaten, die gebleicht und durch Neutralisation auf den pH-Bereich von etwa 6 bis 7 in Estersulfonat pasten überführt werden. In dieser Form haben sie eine große Bedeutung als oberflächenaktive Mittel für Wasch- und Reinigungszwecke.

Trotz optimaler technischer Sulfonierungsbedingungen werden bei der Herstellung von Estersulfonaten häufig Chargen erhalten, die entweder nicht oder nur schwer bleichbar und damit begrenzt technisch einsetzbar sind.

Als Hauptursache für die Bildung dunkelfarbiger Produkte werden Oxidationsprodukte des verwendeten Fettsäureesters (Hydroxy-, Dihydroxy-, Oxoester) verantwortlich gemacht, die zu einem Teil aus der oxidativen Schädigung des Fettsäureanteils bei der Gewinnung und Lagerung des Fettes oder Öles und bei der Herstellung der Alkylester resultieren und die zum anderen Teil im Verlauf der Sulfonierung der Alkylester mit gasförmigem Schwefeltrioxid durch die starke Oxidationswirkung des Schwefeltrioxids gebildet werden.

Durch Umpumpen unter Luftabschluß oder Destillation über Borsäure läßt sich die Bildung von Oxo-Nebenprodukten bei der Aufarbeitung der Triglyceride zu den gehärteten Fettsäureestern mit technischen Mitteln begrenzen. Bei der Sulfonierung der Alkylester zeigen diese technischen Maßnahmen jedoch nur geringe Wirkung. Die Oxidationswirkung des Schwefeltrioxids läßt sich nach den bekannten Methoden lediglich durch Untersulfierung oder Komplexierung des Schwefeltrioxids herabsetzen. Die Untersulfierung führt jedoch zu verminderten Ausbeuten. Als Komplexierungsmittel sind beispeilsweise Dioxan (M. Suter et al., J. Am. Chem. Soc. 60 (1938), 538-540), Alkylphosphate (A. F. Turbak et al., Ind. Eng. Chem. Prod. Res. Develop. 2 (1963), 229-231) oder Dimethylsulfid (M. Nagayama in Bull. Chem. Soc. Japan 47 (1974), 2473-2475) vorgeschlagen worden. Die genannten Verfahren haben jedoch den Nachteil, daß sie einerseits zu technisch nicht ausreichenden Umsetzungsgraden führen und/oder andererseits mit erheblichen präparativen Schwierigkeiten verbunden sind, so daß sie für eine großtechnische Anwendung ungeeignet sind.

Die Aufgabe der vorliegenden Erfindung bestand zum einen darin, ein verbessertes Verfahren zur Herstellung hellfarbiger alpha-Sulfofettsäurealkylestersalze durch Sulfonieren von Fettsäurealkylestern mit einem Sulfonierungsmittel und nachfolgendes Aufarbeiten zur Verfügung zu stellen. Insbesondere sollte bei der Sulfonierung mit Schwefeltrioxid dessen Oxidationswirkung herabgesetzt werden, ohne daß dadurch die Umsetzungsgrade der Sulfonierung vermindert werden oder andere präparative Schwierigkeiten auftreten.

Es wurde gefunden, daß sich der Zusatz geringer Mengen bestimmter Fettsäureamide zu den Fettsäurealkylestern positiv auf deren Oxidationsstabilität auswirkt, so daß bei der Sulfonierung mit Schwefeltrioxid allenfalls geringe Mengen farbaktiver Oxoverbindungen gebildet und Estersulfonate in einer gleichbleibend guten Farbqualität erhalten werden.

Gegenstand der Erfindung ist ein Verfahren zur Herstellung hellfarbiger alpha-Sulfofettsäurealkylestersalze durch Sulfonieren von Fettsäurealkylestern mit gasförmigem Schwefeltrioxid und nachfolgendes Aufarbeiten, das dadurch gekennzeichnet ist, daß man die Fettsäurealkylester in Gegenwart von 0,25 bis 1,5 mol-% - mol-% bezogen auf Fettsäurealkylester - mindestens eines Fettsäureamids der Formel I

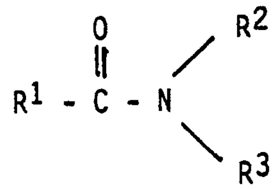 (I)

in der $R^1$ einen geradkettigen Alkylrest mit 5 bis 21 C-Atomen bedeutet, $R^2$ und $R^3$ unabhängig voneinander ein Wasserstoffatom, einen geradkettigen oder verzweigten Alkylrest mit 1 bis 6 C-Atomen darstellen oder $R^2$ und $R^3$ zusammen eine Tetra- oder Pentamethylenkette eines heterocyclischen Ringsystems bilden, in dem gegebenenfalls eine Methylengruppe durch ein Sauerstoffatom, ein Schwefelatom oder eine NH-Gruppe ersetzt sein kann, sulfoniert.

In einer bevorzugten Ausführungsform der Erfindung sulfoniert man die Fettsäurealkylester in Gegenwart von 0,75 bis 1,0 mol-% Fettsäureamid.

Bei den Fettsäureamiden der Formel I handelt es sich um bekannte Substanzen, die nach gängigen Methoden der organischen Synthese hergestellt werden können. Beispiele für geeignete Verbindungen sind Capronsäure-N,N-dimethylamid, Caprylsäure-n-butylamid, Caprinsäure-N,N-diethylamid, Laurinsäure-N,N-dimethylamid, Laurinsäuremorpholid, Myristinsäure-i-propylamid, Myristinsäure-N,N-dimethylamid, Myristinsäurepyrrolidid, Palmitinsäureamid, Palmitinsäure-i-butylamid, Palmitinsäure-N,N-dimethylamid, Stearinsäureamid, Stearinsäuremethylamid, Stearinsäure-N,N-dimethylamid, Stearinsäure-n-propylamid, Stearinsäure-N,N-di-n-propylamid, Stearinsäure-i-propylamid, Stearinsäure-n-butylamid, Stearinsäure-N,N-di-n-butylamid, Stearinsäure-n-pentylamid, Stearinsäure-N,N-di-n-hexylamid, Stearinsäure-i-butylamid, Stearinsäure-N,N-di-i-butylamid, Stearinsäurepyrrolidid, Stearinsäurepiperidid, Stearinsäuremorpholid, Behensäureamid und Behensäure-N,N-dimethylamid.

Die Fettsäureamide der Formel I können als Einzelverbindungen oder in Form von Gemischen mehrerer Einzelverbindungen eingesetzt werden. In einer bevorzugten Ausführungsform der Erfindung werden den Fettsäurealkylestern Fettsäureamide der Formel I zugesetzt, deren Reste $R^1$ 15 bis 17 C-Atome besitzen, wobei die Reste $R^2$ und $R^3$ dieser Fettsäureamide wiederum bevorzugt aus geradkettigen Alkylresten bestehen.

Für die Sulfonierung nach dem erfindungsgemäßen Verfahren kommen Ester von gesättigten Fettsäuren mit niederen aliphatischen Alkoholen in Betracht, insbesondere $C_1$-$C_4$-Alkylester von gesättigten Fettsäuren mit 6 bis 22 C-Atomen, vorzugsweise 8 bis 18 C-Atomen. Die Fettsäurereste dieser Ester stammen aus natürlichen Fetten und Ölen und sind deshalb ausschließlich oder zumindest überwiegend geradkettig. Die Alkylreste der Fettsäureester stammen aus geradkettigen oder verzweigten aliphatischen Alkoholen mit 1 bis 4 C-Atomen. Vorzugsweise werden Methylester als Ausgangsmaterial für die Sulfonierung eingesetzt.

Die Fettsäurealkylester werden nach bekannten Verfahren aus Fetten und Ölen natürlichen Ursprungs entweder über Fettspaltung und nachfolgende Veresterung der freien Fettsäuren mit niederen Alkanolen oder über die Umesterung der natürlichen Triglyceridgemische mit niederen Alkanolen erhalten. Sie sollen außer der alpha-ständigen Methylengruppe keine weiteren sulfonierbaren oder sulfatierbaren Gruppen, insbesondere keine Doppelbindungen oder alkoholischen Hydroxylgruppe besitzen. Aus diesem Grund werden die Rohstoffe - entweder die Fette und Öle oder die daraus hergestellten Alkylester - vor dem Sulfonierungsschritt unter den bekannten Bedingungen der Fetthärtung auf Jodzahlwerte unterhalb von 1, vorzugsweise unterhalb von 0,5, hydriert. Im Rahmen der Erfindung ist es grundsätzlich möglich, individuelle Fettsäurealkylester, beispielsweise Methylstearat als Ausgangsmaterial für die alpha-Sulfonierung mit Schwefeltrioxid einzusetzen. In der großtechnischen Praxis werden jedoch in der Regel Alkylester von Fettsäuregemischen eingesetzt.

Für die Durchführung der Sulfonierung gelten die Bedingungen der einschlägigen bekannten Verfahren zur alpha-Sulfonierung von Fettsäurederivaten, wie sie beispielsweise in der DE-A-12 46 718 und der DE-A-12 48 645 beschrieben sind. Die Sulfonierung erfolgt mit einem Gemisch aus gasförmigem Schwefeltrioxid und einem Inertgas, das üblicherweise 2 bis 8 Vol.-% Schwefeltrioxid enthält. Das Molverhältnis von Fettsäurealkylester zu Schwefeltrioxid kann im Bereich von 1 : 1,1 bis 1 : 1,8 liegen, wobei der Bereich von 1 : 1,2 bis 1 : 1,5 bevorzugt ist. Als Inertgas wird in großtechnischen Verfahren vorzugsweise Luft eingesetzt. Die Reaktionstemperatur kann 10 bis 130 °C betragen. Vorzugsweise wird die Sulfonierung bei 60 bis 90 °C durchgeführt. Dabei kann mit konstanter Verfahrenstemperatur oder mit stufenförmiger Temperaturführung gearbeitet werden. Für die kontinuierliche Ausbildung des Sulfonierungsverfahrens haben sich Fallfilm- und Kaskadenreaktoren als zweckmäßig erwiesen.

Unmittelbar nach der Sulfonierungsreaktion wird das erhaltene Reaktionsgemisch bei 70 bis 90 °C über einen Zeitraum von 10 bis 30 Minuten einer Nachreaktion unterworfen (altern). Anschließend wird das Reaktionsprodukt in üblicher Weise gebleicht und neutralisiert.

Die Bleiche kann nach bekannten Verfahren in wässrigem Medium mit Wasserstoffperoxid und/oder Natriumhypochlorit erfolgen. Die Neutralisation des sauren Reaktionsproduktes kann sowohl vor als auch nach der Bleiche durchgeführt werden. Eine Bleiche mit Wasserstoffperoxid vor der Neutralisation ist beispielsweise in der DE-B-11 79 931 beschrieben. Die DE-A-12 34 709 beschreibt eine Kombinationsbleiche, bei der sich an die Behandlung des sauren Reaktionsproduktes mit wässrigem Wasserstoffperoxid die Neutralisation des teilweise gebleichten Sulfonierungsproduktes anschließt, worauf erneut mit Wasserstoffperoxid oder besser mit Natriumhypochlorit ein abschließender Bleichprozess durchgeführt wird. Nach der DE-A-33 19 591 wird das rohe Sulfonierungsprodukt zuerst in einem neutral bis schwach alkalisch eingestellten wässrigen Medium einer Vorbleiche mit Natriumhypochlorit unterworfen, anschließend wird die Salzpaste schwach sauer eingestellt und mit Wasserstoffperoxid oder Wasserstoffperoxid abgebenden Verbindungen nachgebleicht.

Die Neutralisation der sauren Sulfonierungsprodukte wird mit wässrigen Kaliumhydroxidlösungen und vorzugsweise mit wässrigen Natriumhydroxidlösungen durchgeführt. Bei der Bleichung und bei der Neutralisation sind die Verfahrensbedingungen so zu wählen, daß die hier prinzipiell mögliche Esterverseifung weitgehend ausgeschlossen wird.

Im erfindungsgemäßen Verfahren bewirkt der Zusatz von geringen Mengen an Fettsäureamiden der Formel I eine überraschende Erhöhung der Stabilität der Fettsäurealkylester gegenüber der oxidierenden Wirkung des Schwefeltrioxids. Bei der Sulfonierung der Fettsäurealkylester in Gegenwart von Fettsäureamiden der Formel I wird die Bildung von farbaktiven Hydroxy- und Oxofettsäurederivaten offensichtlich weitgehend zurückgedrängt, so daß alpha-Sulfofettsäureestersalze von befriedigender Farbqualität erhalten werden können.

In den nachstehend beschriebenen Beispielen wurde die Sulfonierung des Fettsäurealkylesters und die Aufarbeitung des rohen Sulfonierungsproduktes nach dem folgenden Standardverfahren durchgeführt:

In einem 1 l-Standreaktor mit Heiz- und Kühlmantel und Gaseinleitungsrohr wurde ein Mol Fettsäurealkylester mit dem vorgesehenen Fettsäureamid der Formel I in der vorgesehenen Menge intensiv vermischt und auf 80 °C erwärmt. Anschließend wurde der Fettsäurealkylester mit 96 g (1,2 Mol) Schwefeltrioxidgas (entsprechend einem Molverhältnis von 1 : 1,2) umgesetzt.

Das Schwefeltrioxid wurde durch Erhitzen aus einer entsprechenden Menge Oleum ausgetrieben, mit Stickstoff auf eine Konzentration von 5 Vol.-% verdünnt und im Verlauf von 60 Minuten in den Fettsäurealkylester eingeleitet. Die Temperatur des Reaktionsgemisches wurde dabei unterhalb von 90 °C gehalten.

Nach der Sulfonierung wurde das rohe Sulfierprodukt zunächst 15 Minuten lang einer Nachraktion bei 80 °C im Wasserbad unterworfen und anschließend durch Einrühren in eine Lösung von 44 g (1,1 Mol) Natriumhydroxid in 500 ml Wasser neutralisiert.

Vor der Bestimmung der Farbzahl-Werte wurde das alpha-Sulfofettsäurealkylester-Natriumsalz nach Zugabe von 2 Gew.%, bezogen auf den Anteil an Waschaktivsubstanz, Natriumhypochlorit 120 Minuten lang gebleicht.

Alle Farbzahlmessungen erfolgten photometrisch nach Klett bei einer Aniontensidkonzentration von 5 Gew.-% und bei pH 7 unter Verwendung einer 1 cm-Rundküvette und eines Blaufilters (400 bis 465 nm).

In den angeführten Vergleichsversuchen wurde der Fettsäurealkylester ohne Zusatz von Fettsäureamiden der Formel I sulfoniert.

## Beispiele

### Beispiele 1 und 2

Als Ausgangsmaterial diente ein Talgfettsäuremethylester (Kettenlängenverteilung im Fettsäurerest: 50 Gew.-% $C_{16}$, 50 Gew.-% $C_{18}$; Verseifungszahl 198; Jodzahl 0,2). Dieses Ausgangsmaterial war aus hochwertigem Rindertalg (Fancy-Talg) hergestellt; sein Fettsäureanteil war durch Gewinnung und Aufarbeitung nur geringfügig beeinträchtigt.

Der Talgfettsäuremethylester wurde nach dem weiter oben beschriebenen Standardverfahren unter Zusatz von 0,25 bis 1,50 Mol-% Stearinsäureamid oder Stearinsäure-N,N-Dimethylamid mit Schwefeltrioxid umgesetzt. Die für die resultierenden alpha-Sulfofettsäuremethylester-Natriumsalze gefundenen Klett-Farbzahlen sind in der Tabelle I in Relation zu dem Farbzahlwert eines ohne Fettsäureamidzusatzes erhaltenen Endproduktes wiedergegeben.

Tabelle I

| Sulfonierung in Gegenwart von Stearinsäureamid oder Stearinsäure-N,N-dimethylamid | | | |
|---|---|---|---|
| Beispiel | | mol-% | Klett-Farbzahl |
| 1 | $C_{17}H_{35}CONH_2$ | 0,25<br>0,50<br>0,75<br>1,00<br>1,50 | 24<br>20<br>24<br>21<br>30 |
| 2 | $C_{17}H_{35}CON(CH_3)_2$ | 0,25<br>0,50<br>0,75<br>1,00<br>1,50 | 21<br>18<br>11<br>13<br>18 |
| Vergleich 1 | ohne Zusatz | - | 42 |

Beispiele 2 bis 22

Als Ausgangsmaterial diente ein Talgfettsäuremethylester (Kettenlängenverteilung im Fettsäurerest: 50 Gew.-% $C_{16}$, 50 Gew.-% $C_{18}$; Verseifungszahl 198; Jodzahl 0,9). Dieses Ausgangsmaterial war aus Rindertalg minderer Qualität (A-Talg) hergestellt; es zeigte Schädigungen des Fettsäureanteils durch Gewinnung und Aufarbeitung.

Der Talgfettsäuremethylester wurde nach dem weiter oben beschriebenen Standardverfahren unter Zusatz von jeweils 0,5 und 1,0 mol.-% der in der Tabelle II aufgeführten Fettsäureamide der Formel I mit Schwefeltrioxid umgesetzt und zu den entsprechenden alpha-Sulfofettsäuremethylester-Natriumsalzen aufgearbeitet. Die Klett-Farbzahlen der jeweiligen Endprodukte sind in der Tabelle II im Vergleich zu dem Farbzahlwert eines Endproduktes angegeben, das ohne Zusatz eines Fettsäureamids erhalten worden war.

## Tabelle II

Sulfonierung in Gegenart von Fettsäureamiden der Formel I

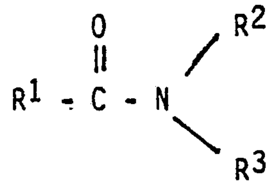

| Beispiel | $R^1$ | $R^2$ | $R^3$ | Klett-Farbzahl 0,5 mol-% | Klett-Farbzahl 1,0 mol-% |
|---|---|---|---|---|---|
| 3 | $C_{17}H_{35}$ | H | H | 93 | 76 |
| 4 | $C_{17}H_{35}$ | $CH_3$ | H | 90 | 74 |
| 5 | $C_{17}H_{35}$ | n-$C_3H_7$ | H | 90 | 81 |
| 6 | $C_{17}H_{35}$ | i-$C_3H_7$ | H | 100 | 85 |
| 7 | $C_{17}H_{35}$ | n-$C_4H_9$ | H | 105 | 87 |
| 8 | $C_{17}H_{35}$ | i-$C_4H_9$ | H | 108 | 91 |
| 9 | $C_5H_{11}$ | $CH_3$ | $CH_3$ | 108 | 82 |
| 10 | $C_{11}H_{23}$ | $CH_3$ | $CH_3$ | 110 | 82 |
| 11 | $C_{13}H_{27}$ | $CH_3$ | $CH_3$ | 109 | 79 |
| 12 | $C_{15}H_{31}$ | $CH_3$ | $CH_3$ | 110 | 75 |
| 13 | $C_{17}H_{35}$ | $CH_3$ | $CH_3$ | 88 | 68 |
| 14 | $C_{21}H_{43}$ | $CH_3$ | $CH_3$ | 108 | 85 |
| 15 | $C_{17}H_{35}$ | n-$C_3H_7$ | n-$C_3H_7$ | 95 | 79 |
| 16 | $C_{17}H_{35}$ | i-$C_3H_7$ | i-$C_3H_7$ | 98 | 78 |
| 17 | $C_{18}H_{35}$ | n-$C_4H_9$ | n-$C_4H_9$ | 105 | 87 |
| 18 | $C_{17}H_{35}$ | i-$C_4H_9$ | i-$C_4H_9$ | 99 | 79 |
| 19 | $C_{17}H_{35}$ | $-(CH_2)_4-$ | | 97 | - |
| 20 | $C_{17}H_{35}$ | $-(CH_2)_5-$ | | 103 | 75 |
| 21 | $C_{17}H_{35}$ | $-(CH_2)_2-O-(CH_2)_2-$ | | 86 | 71 |
| | ohne Zusatz | | | 163 | |

**Ansprüche**

1. Verfahren zur Herstellung hellfarbiger alpha-Sulfofettsäurealkylestersalze durch Sulfonieren von Fettsäurealkylestern mit gasförmigem Schwefeltrioxid und nachfolgendes Aufarbeiten, dadurch gekennzeichnet, daß man die Fettsäurealkylester in Gegenwart von 0,25 bis 1,5 mol-% - mol-% bezogen auf Fettsäurealkylester - mindestens eines Fettsäureamids der Formel I

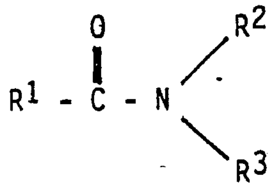

(I)

in der $R^1$ einen geradkettigen Alkylrest mit 5 bis 21 C-Atomen bedeutet, $R^2$ und $R^3$ abhängig voneinander ein Wasserstoffatom, einen geradkettigen oder verzweigten Alkylrest mit 1 bis 6 C-Atomen darstellen oder $R^2$ und $R^3$ zusammen eine Tetra- oder Pentamethylenkette eines heterocyclischen Ringsystems bilden, in dem gegebenenfalls eine Methylengruppe durch ein Sauerstoffatom, ein Schwefelatom oder eine NH-Gruppe ersetzt sein kann, sulfoniert.

2. Verfahren nach Anspruch 1, dadurch gekennzeichnet, daß man in Gegenwart von 0,75 bis 1,0 mol-% Fettsäureamid sulfoniert.

3. Verfahren nach mindestens einem der Ansprüche 1 und 2, dadurch gekennzeichnet, daß in der Formel I $R^1$ einen Alkylrest mit 15 bis 17 C-Atomen bedeutet.

4. Verfahren nach Anspruch 3, dadurch gekennzeichnet, daß in der Formel I die Reste $R^2$ und/oder $R^3$ geradkettige Alkylreste sind.

5. Verfahren nach mindestens einem der Ansprüche 1 bis 4, dadurch gekennzeichnet, daß man $C_1$-$C_4$-Alkylester von gesättigten Fettsäuren mit 6 bis 22 C-Atomen sulfoniert.

6. Verfahren nach Anspruch 5, dadurch gekennzeichnet, daß man Fettsäuremethylester sulfoniert.

Europäisches Patentamt

# EUROPÄISCHER RECHERCHENBERICHT

Nummer der Anmeldung

EP 89 11 8254

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | FR-A-1 336 901 (HENKEL)<br>* Anspruch 1 *<br>--- | | C 07 C 303/06<br>C 07 C 309/17 |
| A | DE-A-2 516 691 (LION FAT & OIL CO.)<br>--- | | |
| D,A | DE-A-1 234 709 (HENKEL)<br>--- | | |
| D,A | DE-A-1 246 718 (HENKEL)<br>--- | | |
| A | DE-A-1 258 864 (HENKEL)<br>----- | | |

| RECHERCHIERTE SACHGEBIETE (Int. Cl.5) |
|---|
| C 07 C 303/00<br>C 07 C 309/00 |

Der vorliegende Recherchenbericht wurde für alle Patentansprüche erstellt

| Recherchenort | Abschlußdatum der Recherche | Prüfer |
|---|---|---|
| DEN HAAG | 10-01-1990 | ZAROKOSTAS K. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)